# EUROPEAN PATENT APPLICATION

(11) **EP 2 293 059 A2**
(43) Date of publication of application: **09.03.2011**
(21) Application number: 09754302.9
(22) Date of filing: 28.05.2009
(51) Int. Cl.: G01N 30/06, G01N 33/34, G01N 33/497

(54) **PROFILES OF VOLATILE HYDROCARBON COMPOUNDS, MARKERS OF DEGRADATION/AGEING OF CELLULOSIC MATERIAL AND DISEASE MARKERS**

(30) Priority: 28.05.2008 PT 08104077
(71) Applicant: Universidade Nova de Lisboa, 1099-085 Lisboa (PT)
(72) Inventor: MENDES SARDÃO MONTEIRO GASPAR, Elvira Maria, P-2685-212 Portela LRS (PT); FOLGADO DE LUCENA, Ana Filipa, P-2825-053 Caparica (PT)
(74) Representative: Ferreira, Maria Silvina
(86) International application number: PCT/IB2009/052256
(87) International publication number: WO 2009/144677

(57) **Abstract**

The present invention relates to profiles (significant set) of volatile organic compounds, VOCs, namely hydrocarbons between 2 and 24 carbon atoms (C₂-C₂₄), using solid phase microextraction (SPME) and high resolution gas chromatography coupled to mass spectrometry (GC-MS) as methodology. The present invention provides the identification and characterization of profiles of cellulosic material, markers of degradation and/or ageing of cultural heritage, or the identification and characterization of profiles of biological fluids, markers of pathology. The present invention provides the identification and characterization of the hydrocarbons, chemical components of the profiles, markers of chemical degradation of cellulosic polymer or of lipid peroxidation due to pathology.

## Description

### Technical Field of the Invention

The present invention relates to the identification and characterization of profiles (significant set) of hydrocarbons containing between 2 and 24 carbon atoms (C₂-C₂₄), the so-called volatile organic compounds (VOCs); the chemical composition of the hydrocarbon profiles constitutes a marker of ageing of the cellulosic material, allowing the identification and characterization of the preservation or degradation state of the cellulose-based material of cultural heritage; the chemical composition of the volatile hydrocarbon profiles of biological samples constitutes a marker of the occurrence of lipid peroxidation. The present invention provides the identification and characterization of the profiles and their chemical components, volatile hydrocarbons, markers of cellulosic polymer chemical degradation or of lipid peroxidation due to pathology.

The analysis of historical objects belonging to cultural heritage is often difficult due to restricted sampling / collecting process. The historical value of the objects and their integrity usually superimpose on the scientific insight gained with the destructive sampling of the articles for chemical analysis. When analysing paper and other materials of cellulosic nature, the parameter of major interest is essentially the pH. The degradation process depends on this parameter: in an acidic environment, cellulose degrades through acid catalysed-hydrolysis, while in a moderately alkaline environment it degrades through self-oxidation. The latter process is much slower, the estimated average degradation time for moderately alkaline paper produced before 1850 being a few millenniums. Acidic paper produced between 1850 and 1990 is expected not to survive another century, corresponding to the total loss of documents belonging to this time period. The catastrophic circumstance of the process is the estimation that over 70% of the documents stored in western libraries are acidic [1].

### Previous art

The development of non-destructive analytical methods usable in cultural heritage has been recently focused on techniques for volatile organic compound (VOC) analysis. The relevance of volatiles and the fact that organic volatile compounds are an excellent source of information are known since 1982 [2], using the HPLC and IEC techniques. The dawn of the solid phase microextraction (SPME) [3-5] sampling technique made it possible to analyse volatile compounds emitted from matrices of cultural heritage. By combining SPME with gas chromatography coupled to mass spectrometry (GC-MS), analyses have been made involving matrices of cultural heritage, including material of cellulosic nature [6,7]. The target compounds, however, have been furfural and acetic acid [6,7], carboxylic acids, aldehydes, ketones, benzene and derivatives, naphthalene and derivatives, and some hydrocarbons [8,9,10].

The present invention provides the surprising innovation of identifying profiles of volatile hydrocarbons in objects of cellulosic nature, establishing the relation between the hydrocarbon profile, the dating and/or degradation/preservation state of the objects, which have never been done before. Another surprising innovation is the establishment, not of one or two organic compounds as heretofore but of a set of hydrocarbons as markers of degradation and/or ageing through non-destructive analysis of the objects of cultural heritage, keeping them absolutely intact, namely books, textiles, and cloths, using solid phase microextraction coupled to gas chromatography and mass spectrometry (SPME-GC-MS). It should also be noticed that the profiles are especially useful, having the potential to contribute, as analytical tool, to the identification and characterization of biological fluids and exhaled air, allowing the identification of profiles of volatile hydrocarbons as markers of lipid peroxidation due to pathology.

This invention constitutes a new, simple and fast means of determining new markers, profiles, of cellulosic polymers chemical degradation, providing the establishment of the appropriate conditions for the preservation of cultural heritage.

The process has the potential of being automated/robotized, allowing the automated analysis of samples of historic objects, and likewise of biological samples, for the identification of markers of degradation/ageing of the cellulosic material or markers of pathology.

### Detailed Description of the Invention

The present invention relates to profiles of volatile organic compounds, VOCs, namely hydrocarbons between 2 and 24 carbon atoms (C₂-C₂₄), using solid phase microextraction (SPME) and high resolution gas chromatography coupled to mass spectrometry (GC-MS) as methodology.

In a preferred embodiment of the invention, the method consists firstly in sample preparation, collecting the volatile compounds by contacting the solid phase microextraction (SPME) fibre, loaded into the syringe/holder (Supelco), with the materials to be analysed, the latter being kept in inert, closed containers in order to allow collecting and concentrating the target compounds, volatile hydrocarbons (C₂-C₂₄). The fibre is made of polydimethylsiloxane (POMS, Supelco) or a derivative thereof, and the contact time is in the range of 0.5 - 36 h. The analysis is made by means of high resolution gas chromatography coupled to mass spectrometry (GC-MS). Before use, the fibres were conditioned at 260/270°C for 5 - 60 min. The fibre was initially conditioned and the bag blank was performed; the fibre blank was also performed before each analysis.

### Experimental Part

### Equipment and general reagents

The grades of used hydrocarbons are of the highest purity level commercially available. The separations were made using high resolution gas chromatography coupled to mass spectrometry (HRGC-MS) using a quadruple TRACE GC-MS device (Finnigan) equipped with a split-splitless injector. The injector temperature was 230°C. The splitless time was 2.5 min and the split ratio was 1:30. The capillary column was BPX5 (30 m x 0.25 mm x 1 µm film thickness). Helium was used as carrier gas with a flow rate of 1 mLmin⁻¹. The MS analyses were made in full scan mode in the range of 30 - 460 amu. A scan ratio of 3.46 scan s⁻¹ was used. Ionization: EI, 70 eV, ionic source and interface at 200°C and 250°C, respectively. Data were collected and processed by means of the software Xcalibur. The column temperature program was 35°C for 10 min, increased to 150°C at 3°Cmin⁻¹, increased to 220°C at 10°Cmin⁻¹, increased to the final temperature of 240°C at 5°Cmin⁻¹, then kept constant for 10 min.

### Examples

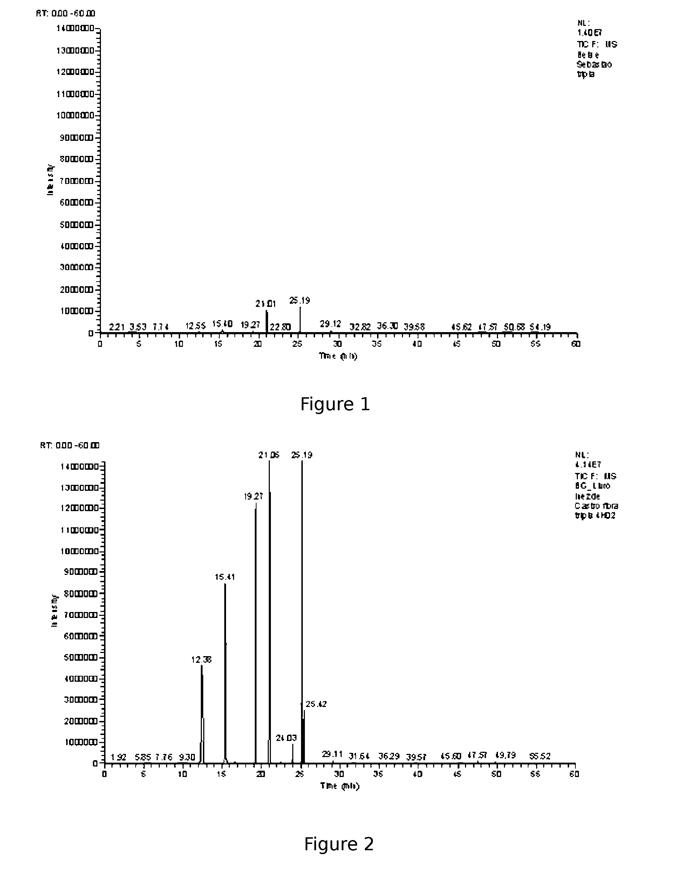

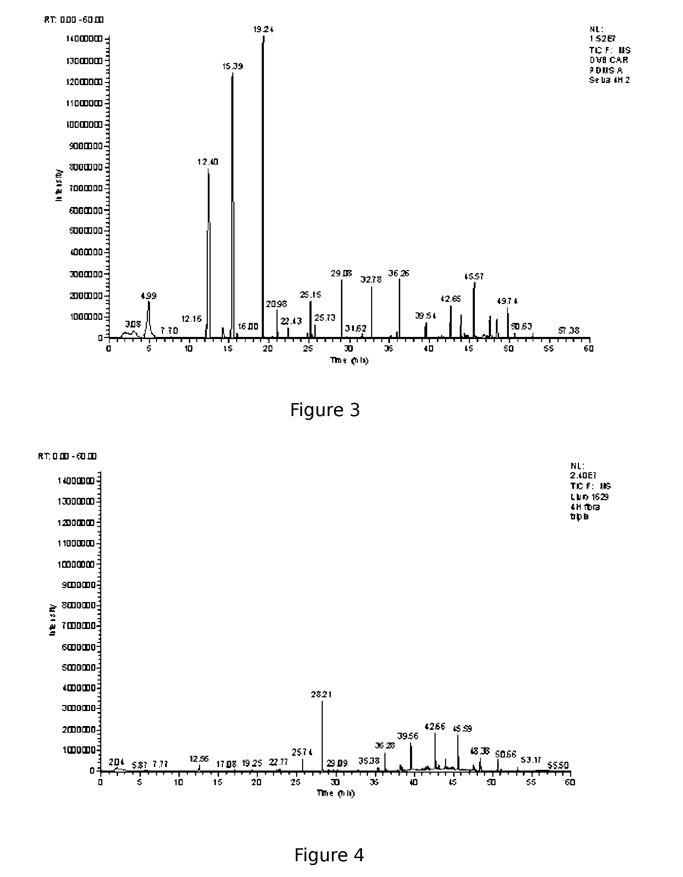

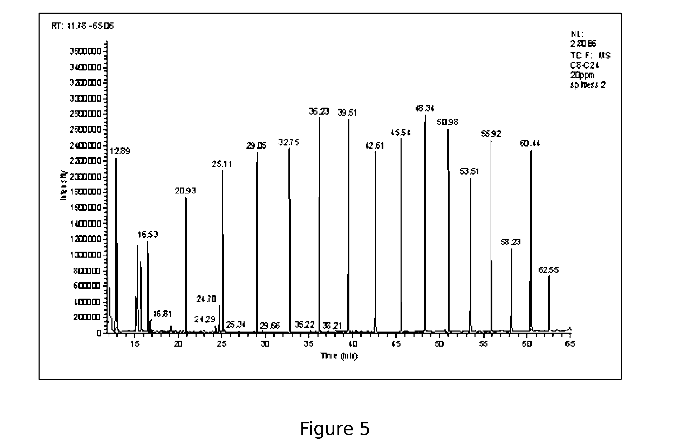

Example 1: Determination of the profile of volatile hydrocarbons in a recent book: chromatographic analysis of a recent book (2003) - separation and identification of volatile hydrocarbons.
   During the sample collecting process, the book was kept isolated in a zip bag. The fibre was introduced between the pages of the book and was exposed for 0.5 - 36 h at room temperature. The fibre was collected before conducting the analysis, being then analysed by GC-MS using the conditions already described.
   The profile of volatile hydrocarbons, result of the obtained separations is shown in Figure 1.
Example 2: Determination of the profile of hydrocarbons in a degraded old book: chromatographic analysis of a degraded old book (1900) - separation and identification of volatile hydrocarbons.
   As in example 1, the book was kept isolated in a zip bag. The fibre was introduced between the pages of the book and was exposed for 0.5 - 36 h at room temperature. The fibre was collected before conducting the analysis, being then analysed by GC-MS using the conditions described above.
   The profile of volatile hydrocarbons as a result of the obtained separations is shown in Figure 2.
Example 3: Determination of the profile of hydrocarbons in an old book subject to degradation: chromatographic analysis of an old book (~1930) subject to degradation - separation and identification of volatile hydrocarbons.
   The book was likewise kept isolated in a zip bag. The fibre was introduced between the pages of the book and was exposed for 0.5 - 36 h at room temperature. The fibre was collected before conducting the analysis, being then analysed by GC-MS using the conditions already described. Figure 3 shows the profile of an old book subject to degradation as a result of the separations obtained for the volatile hydrocarbons.
Example 4: Determination of the profile of hydrocarbons in an ancient book: chromatographic analysis of a preserved ancient book (1629) - separation and identification of volatile hydrocarbons.
   The fibre was introduced between the pages of the zip bag-isolated book and was exposed for 0.5 - 36 h at room temperature. The fibre was collected before conducting the analysis, being then analysed by GC-MS using the conditions already described. Figure 4 presents the profile of a preserved ancient book as a result of the separations obtained for the volatile hydrocarbons.
Example 5: Determination of the chromatographic profile of standards, hydrocarbons with C₈ to C₂₄ carbon atoms.
   The SPME fibre was inserted for 30 s into a sealed vial containing the hydrocarbon standards, which had been left in equilibrium for 30 min at room temperature. The fibre was collected before conducting the analysis, being then analysed by GC-MS using the conditions already described. Figure 5 presents the profile of standard volatile hydrocarbons with C₈ to C₂₄ carbon atoms.

### Description of the Figures

Figure 1 - Profile of volatile hydrocarbons in a recent book (2003); rₜ=21.01 min: C₁₀; rₜ=25.19 min: C₁₁.
Figure 2 - Profile of volatile hydrocarbons in a degraded old book (1900); rₜ=21.05 min: C₁₀; rₜ=25.19: C₁₁.
Figure 3 - Profile of volatile hydrocarbons in an old book (~1930) subject to degradation; C₁₀: rₜ=20.98; C₁₁: rₜ=25.15; C₁₂: rₜ=29.08; C₁₃: rₜ=32.78; C₁₄: rₜ=36.26; C₁₅: rₜ=39.54; C₁₆: rₜ=42.65; C₁₇: rₜ=45.57; C₁₈: rₜ=48.34 min.
Figure4 4 - Profile of volatile hydrocarbons in a preserved ancient book (1629); C₁₉: rₜ=36.28; C₁₅: rₜ=39.56; C₁₆: rₜ=42.66; C₁₇: rₜ=45.59; C₁₈: rₜ=48.36; C₁₉: rₜ=50.66; C₂₀: rₜ=53.17 min.
Figure 5 - Retention times for the standards of volatile hydrocarbons: C₈: rₜ=12.89; C₉: rₜ=16.53; C₁₀: rₜ=20.93; C₁₁: rₜ=25.11; C₁₂: rₜ=29.05; C₁₃: rₜ=32.75; C₁₄: rₜ=36.23; C₁₅: rₜ=39.51; C₁₆: rₜ=42.61; C₁₇: rₜ=45.54; C₁₈: rₜ=48.34; C₁₉: rₜ=50.96; C₂₀: rₜ=53.51; C₂₁: rₜ=55.92; C₂₂: rₜ=58.23; C₂₃: rₜ=60.44, and C₂₄: rₜ=62.55 min.

Figures 1 to 4 show the obtained results: the profiles of recent books, of old books kept in poor preservation conditions, and old books subject to "normal" degradation due to ageing, considering their age and the time span; they show the obtained separations and the identification of volatile hydrocarbons that constitute the different profiles.

Figure 5 shows the retention times obtained during the separation of some hydrocarbon standards that were analysed using HS-SPME-GC-MS.

In each case, the profile of the compounds is very characteristic, being chemically compatible with the nature of the analysed objects: recent books (Figure 1) have almost no volatile hydrocarbons, a result which is compatible with the lack of degradation of the cellulosic material. The profile of an ancient book (1629) (Figure 4) shows that, in spite of being composed by cotton cellulose and not wood cellulose, the profile contains a cluster of long chain hydrocarbons, C₁₂-C₂₀, with the maximum of the profile involving the C₁₆-C₁₇ hydrocarbons. It confirms the occurrence of age "degradation", the cellulose degradation due to time course. The profile of a somewhat old but completely degraded book, actually falling into pieces due to the presence of moisture and an acidic environment (Figure 2), shows that the profile includes the presence of hydrocarbons between C₈ and C₁₁, indicating that in the profile composition the carbon chain decreases with increasing degradation, the intensity of these compounds revealing their relative quantitative presence. Figure 3 shows a profile indicating the presence of long chain hydrocarbons due to ageing, and compounds containing between C₈-C₁₁ due to cellulose degradation.

Using books from our historic cultural heritage as example, the results show easily recognisable profiles (a simple visual analysis being sufficient, not being even required to apply statistical methods defining profiles and markers) of volatile hydrocarbons, markers and identifiers of dating (although a rough one) and/or ageing (due to normal time course) and/or early degradation of the cellulosic material due to inadequate preserving conditions. Similar results, that is to say characteristic profiles of volatile hydrocarbons, were obtained with exhaled air, showing that there are profiles that act as markers of the occurrence of lipid peroxidation due to pathology.

### Bibliographic References

Baranski, A. Konieczna-Molenda, A., Lagan, J.M., Proniewicz, L.M., Restaurator 24, 36 (2003).
Shafizadeh, F., J. Anal. Appl. Pyrolysis 3, 283 [1982). Pawliszyn, J., Trends Anal. Chem. 14, 113 (1995).
Prosen, H., Zupan 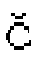 i 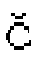 -Kralj, L., Trends Anal. Chem. 18, 272 (1999).
Vas, G., Vékey, K., J. Mass Spectrom. 39, 233 (2004)
Godoi, A.F.L., Van Vaeck, L., Van Grieten, R., J. Chromatogr. A 1067, 331 (2005).
Strli 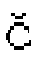 , M, Cigi 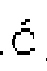 , I.K. Kolar, J., Bruin, G., Pihlar, B., Sensors 7, 3136 (2007).
Lattuati-Derieux, A., Bonnassies-Termes, S., Lavédrine, B., J. Chromatogr. A 1026, 9 (2004).
Lattuati-Derieux, A., Bonnassies-Termes, S., Lavédrine, B., J. Cult. Herit. 7, 123 (2006).
Wenzl, T., Lankmayr, E.P., J. Chromatogr. A 897, 269 (2000).

## Claims

1. Profiles of volatile hydrocarbons containing between 2 and 24 carbon atoms (C₂-C₂₄), **characterized in that** the head-space (HS--SPME) or direct immersion (DI-SPME) SPME methodology, DI/HS-SPME-GC-MS, is used employing Polydimethylsiloxane (PDMS) fibre and/or PDMS-containing derived fibre, and various chromatographic columns, including the chiral chromatographic separation ones.

2. Profiles of volatile hydrocarbons (C₂-C₂₄) according to claim 1, **characterized in that** polymeric chemical degradation of cellulosic materials and lipid peroxidation in bioprocesses are determined.

3. Profiles of volatile hydrocarbons (C₂-C₂₄) according to claims 1 and 2, **characterized in that** samples of cultural heritage are analysed for identifying the occurrence of degradation and the preservation conditions of the objects, or biological samples are analysed for identifying the occurrence of pathology / disease, in an automated / robotized / online way.

4. Use of profiles of volatile hydrocarbons (C₂-C₂₄) according to claims 1 and 2, **characterized in that** degradation of cultural heritage and preservation conditions of cellulosic materials from books, textiles, and cloths are detected.

5. Use of profiles of volatile hydrocarbons (C₂-C₂₄) according to claims 1 and 3, **characterized in that** changes in human metabolism are identified by means of primary detection of pathology and/or oncological disease in biological fluids and exhaled air.

6. Analysis process according to claims 1 to 3, **characterized in that** markers of ageing/polymeric (cellulosic) degradation are detected by analysing the profiles of hydrocarbons in books, textiles, and cloths.
